(19) Europäisches Patentamt

European Patent Office

Office européen des brevets

(11) **EP 1 347 785 B1**

(12) **EUROPÄISCHE PATENTSCHRIFT**

(45) Veröffentlichungstag und Bekanntmachung des Hinweises auf die Patenterteilung:
**14.07.2004   Patentblatt 2004/29**

(21) Anmeldenummer: **01273442.2**

(22) Anmeldetag: **03.12.2001**

(51) Int Cl.[7]: **A61L 2/18**, A61F 2/24

(86) Internationale Anmeldenummer:
**PCT/DE2001/004494**

(87) Internationale Veröffentlichungsnummer:
**WO 2002/058745 (01.08.2002 Gazette 2002/31)**

(54) **VERFAHREN ZUR KONSERVIERUNG VON BIOLOGISCHEN PROTHESEN, KONSERVIERTE BIOLOGISCHE PROTHESE UND KONSERVIERUNGSLÖSUNG**

METHOD FOR CONSERVING BIOLOGICAL PROTHESES, CONSERVED BIOLOGICAL PROTHESES AND CONSERVING SOLUTIONS

PROCEDE POUR CONSERVER DES PROTHESES BIOLOGIQUES, PROTHESES BIOLOGIQUES CONSERVEES ET SOLUTION DE CONSERVATION

(84) Benannte Vertragsstaaten:
**AT BE CH CY DE DK ES FI FR GB GR IE IT LI LU MC NL PT SE TR**

(30) Priorität: **06.12.2000   DE 10060660**

(43) Veröffentlichungstag der Anmeldung:
**01.10.2003   Patentblatt 2003/40**

(73) Patentinhaber:
• **Frey, Rainer**
  **82319 Starnberg (DE)**
• **Wachauf, Gerhard**
  **80539 München (DE)**

(72) Erfinder:
• **BARBARASH, Leonid**
  **650065, Kemerovo (RU)**
• **JOURAVLEVA, Irina**
  **650065, Kemerovo (RU)**
• **NOVIKOVA, Svetlana**
  **123154, Moskau (RU)**

(74) Vertreter: **Walcher, Armin**
  **Louis, Pöhlau, Lohrentz & Segeth**
  **Postfach 3055**
  **90014 Nürnberg (DE)**

(56) Entgegenhaltungen:
WO-A-92/09309        WO-A-97/32472
WO-A-98/46288        US-A- 4 806 595
US-A- 5 880 242

• **LOHRE J M ET AL: "EVALUATION OF EPOXY ETHER FIXED BOVINE ARTERIAL GRAFTS FOR MUTAGENIC POTENTIAL" ASAIO JOURNAL, J.B.LIPPINCOTT CO.,HAGERSTOWN,MD, US, Bd. 39, Nr. 2, 1. April 1993 (1993-04-01), Seiten 106-113, XP000411933 ISSN: 1058-2916**
• **CHANDA J ET AL: "In vitro and in vivo calcification of vascular bioprostheses" BIOMATERIALS, ELSEVIER SCIENCE PUBLISHERS BV., BARKING, GB, Bd. 19, Nr. 18, September 1998 (1998-09), Seiten 1651-1656, XP004161436 ISSN: 0142-9612**
• **SCHMIDT C E ET AL: "Acellular vascular tissues: natural biomaterials for tissue repair and tissue engineering" BIOMATERIALS, ELSEVIER SCIENCE PUBLISHERS BV., BARKING, GB, Bd. 21, Nr. 22, 15. November 2000 (2000-11-15), Seiten 2215-2231, XP004210279 ISSN: 0142-9612**

**Beschreibung**

Die Erfindung betrifft ein Verfahren zur Konservierung von biologischen Prothesen sowie gemäß diesem Verfahren hergestellte biologische Prothesen. Weiterhin betrifft die Erfindung eine Konservierungslösung zur Konservierung biologischer Prothesen.

Biologische Prothesen können aus Körperbestandteilen von Menschen und Tieren hergestellt werden. Beispielsweise werden Herzklappen, Arterien, etc. von Rindern oder Schweinen als Implantate beim Menschen verwendet.

Allerdings müssen die biologischen Prothesen vor der Implantation in den Organismus des Menschen chemisch behandelt sein. Das Verfahren der Behandlung muß gewährleisten:

1) Das Fehlen einer Immunogenität (dies gilt gleichermaßen für heterologe und allogene Gewebe);

2) Die Sterilität des Implantates;

3) Hohe Festigkeits-, Elastizitäts- und Deformationseigenschaften des biologischen Materials;

4) Hohe Biokompatibilität, deren Hauptparameter, bezogen auf die biologischen Prothesen, das Fehlen von Kalzifikation und Thrombosen bei lang andauernder Verwendung im Empfängerorganismus ist.

Seit mehr als 30 Jahren stellt Glutaraldehyd das Hauptkonservierungsmittel für Herz-Kreislauf-Bioprothesen (biologische Prothesen) dar. Glutaraldehyd reagiert mit den Aminogruppen des Lysins und des Hydroxylysins. Als Ergebnis dieser Reaktionen bilden sich chemische Bindungen, die vor allem durch Schiffsche Basen oder Pyridinbasen vertreten sind. Glutaraldehyd gewährleistet eine zuverlässige Sterilität und die Unterdrückung antigener Eigenschaften des biologischen Materials.

Glutaraldehyd macht allerdings das Gewebe steif und hydrophob, die Oberfläche nimmt ein grobes ungeordnetes Relief an. Außerdem verfügen die chemischen Verbindungen von Glutaraldehyd und Kollagen in ihrer Struktur über Liganden für die Komplexbildung mit Kalziumkationen. Diese Komplexe werden wohl in der Folge zu Zentren der Hydroxylapatitkristallisation. In der Gesamtheit führt das dazu, daß der Glutaraldehyd einen negativen Einfluß auf die thromboseresistenten Eigenschaften der Gewebe hat und die Kalzifikation der biologischen Materialien hervorruft.

Um den negativen Einfluß von Glutaraldehyd auf das biologische Material zu beseitigen, wurden Verfahren zur zusätzlichen chemischen Modifikation von biologischen Prothesen entwickelt.

Für die Erhöhung der Thromboseresistenz wurde z.B. Heparin verwendet (US 3,988,782); für die Prophylaxe der Kalzifikation wurden Aminodiphosphonate verwendet (US 4,553,974). Allerdings haben diese Methoden nicht die erwartete Wirkung, da der negative Einfluß des Glutaraldehyds als Hauptkonservierungsmittel auf das Gewebe zu groß ist.

Einegrößere Wirkung kann erzielt werden durch Ersatz des Hauptkonservierungsmittels gegen ein Vernetzungsmittel, in dessen Strukturformel keine Aldehydgruppe vorhanden ist.

Bekannt sind Verfahren der Konservierung von biologischen Prothesen unter Nutzung individueller polymerer (US 4,806,595, US 5,080,670) und nichtpolymerer (US 5,880,242, RU 2,008,767) Epoxidverbindungen.

Epoxidverbindungen sind wirksame Vernetzungsmittel, sie gewährleisten die Festigkeit und Elastizität des biologischen Materials, hemmen die Kalzifikation der biologischen Prothesen und verfügen über ausgeprägte antigendepressive Eigenschaften.

Zur Verbesserung der Thromboseresistenz des Gewebes kann eine zusätzliche Modifikation mit Heparin erfolgen (US 4,806,595, RU 2,008,767). Die Nachteile dieser Verfahren sind die folgenden:

1) Maskier-Effekt ("masking" effect), der für nichtpolymere oder polymere Diepoxide charakteristisch ist, wenn eine der Epoxygruppen mit Kollagen reagiert, die andere aber ohne Verbindung bleibt, d.h. nicht reagiert. Auf der einen Seite zeigen diese ungebundenen, d.h. freien Epoxygruppen einen zytotoxischen Effekt, auf der anderen Seite führen diese zu einer ungenügenden Dichte an Quervernetzung, was einen negativen Einfluß auf die Festigkeitseigenschaften des Gewebes hat.

2) Der Einsatz von individuellen Polymerepoxiden, in deren Strukturformel mehr als 2 Epoxidgruppen enthalten sind, vermindert den Maskier-Effekt, verschlechtert aber die Quervernetzung und vergrößert den Gehalt an freien Epoxidgruppen im Gewebe. Ohne eine auf das Schließen bzw. Umsetzen dieser Gruppen gerichtete zusätzliche Modifikation, erhöht sich auf der einen Seite der zytotoxische Effekt. Auf der anderen Seite ist die Anzahl dieser Epoxidgruppen zu klein für eine Sättigung des biologischen Materials mit Stoffen, die der biologischen Prothese zusätzliche Eigenschaften verleihen (Heparin, antibakterielle Mittel u.a.).

Das aus der US 4,806,595 bekannte Verfahren der Heparinbehandlung von epoxykonservierten biologischen Prothesen umfaßt die Verwendung von Protamin, welches durch eine Epoxidverbindung fest mit dem Kollagen verbunden ist. Auf dem Protamin wiederum fixiert man Heparin, welches, sich von der Oberfläche abhebend, eine antikoagulante Funktion ausübt.

Die Effektivität eines solchen Verfahrens ist jedoch zeitlich begrenzt und erfordert den Einsatz eines Zwischenreagenses, nämlich von Protamin. Die Möglichkeit der Bindung von Protamin mit dem Kollagen - wenn es sich um Kollagen aus dem Bindegewebe der biologischen Prothese handelt - ist ihrerseits limitiert durch die Anzahl freier reaktionsfähiger Gruppen des Konservierungsmittels, da der Hauptteil dieser Gruppen für die Quervernetzung in Anspruch genommen wird.

Aus der US 5,165,919 ist ein Verfahren zur kovalenten Fixierung von Heparin auf medizinischen Implantaten durch die Wechselwirkung von Amino- und Epoxygruppen bekannt. Dieses Verfahren wurde jedoch für polymere Materialien entwickelt und ist für biologische Prothesen nicht anwendbar.

Aufgabe der Erfindung ist es, ein Verfahren zur Konservierung von biologischen Prothesen, die gegenüber Kalzifikation und Thrombosebildung beständig sind sowie über eine erhöhte Festigkeit und Elastizität verfügen, bereitzustellen. Weiterhin ist es eine Aufgabe der Erfindung verbesserte konservierte biologische Prothesen bereitzustellen.

Die der Erfindung zugrundeliegende Aufgabe wird durch ein Verfahren mit den Merkmalen gemäß Patentanspruch 1 gelöst. Bevorzugte Weiterbildungen sind in den Unteransprüchen 2 bis 17 angegeben.

Weiterhin wird die Aufgabe durch eine biologische Prothese gemäß Patentanspruch 18 gelöst. Eine bevorzugte Ausführungsform der biologischen Prothese ist in Unteranspruch 19 angegeben.

Ferner wird die Aufgabe durch Bereitstellung eines Konservierungsmittels für biologische Prothesen gemäß Patentanspruch 20 gelöst. Erfindungsgemäß werden bevorzugt für die Konservierung von biologischen Prothesen Gemische aus polymeren und nichtpolymeren Epoxidverbindungen verwendet, wobei in deren Strukturformel 2 und mehr Epoxidgruppen vorhanden sind.

Bevorzugt wird eine Lösung mit einem Gemisch von wenigstens drei verschiedenen Epoxidverbindungen (Komponenten) verwendet.

Wesentlich im Sinne der Erfindung ist, daß das Antithrombotikum über antithrombotische oder antikoagulierende Eigenschaften verfügt.

Bevorzugt wird im Sinne der Erfindung das Antithrombotikum Heparin verwendet.

Es wurde überraschend herausgefunden,

1) daß sich die Festigkeit der biologischen Prothese durch Querverbindungen erhöht, da die Zugänglichkeit von reaktiven Gruppen von Kollagen in der biologischen Prothese für eine Wechselwirkung mit verschiedenen Konservierungsmitteln, die über verschiedene Moleküllängen verfügen, gewährleistet ist. Dies erhöht die Festigkeit des biologischen Materials und gewährleistet eine bessere Antigendepression;

2) daß die Verwendung von Verbindungen mit mehr als 2 Epoxidgruppen in dem Gemisch von Epoxidverbindungen die Gewinnung von biologischem Material mit einer vorgegebenen Anzahl an freien Epoxygruppen ermöglicht;

3) daß nachfolgend auf diesen Gruppen Heparin immobilisiert werden kann zur Verleihung einer Thromboseresistenz. Eine Immobilisierung von Heparin im Komplex mit Acetylsalicylsäure verstärkt den Thromboseresistenzeffekt.

Nach Konservierung und Modifizierung mit Heparin kann man die so hergestellten biologischen Prothesen in einer Lösung einer beliebigen, die Sterilität gewährleistenden Verbindung aufbewahren. Vorzugsweise werden Epoxidverbindungen verwendet, um mögliche zusätzliche chemische Reaktionen zu vermeiden.

Gegenstand der vorliegenden Erfindung ist somit zum einen ein Verfahren zur Konservierung von biologischem Material, welches insbesondere für die Gewinnung von Herz- und Blutgefäßklappenprothesen verwendet werden kann. Zum anderen ist Gegenstand der Erfindung die Bereitstellung der gemäß dem erfindungsgemäßen Verfahren konservierten biologischen Prothesen.

Erfindungsgemäß werden bevorzugt für die Konservierung anstelle individueller Epoxidkonservierungsmittel Mischungen aus polymeren und nichtpolymeren Epoxidverbindungen mit einer unterschiedlichen Anzahl von Epoxidgruppen (2 und mehr) verwendet. Die Zusammensetzung der Mischung kann in Abhängigkeit von der Art des biologischen Materials und den Zielen einer nachfolgenden chemischen Modifikation variiert werden.

Bevorzugt ist die Epoxidgruppe in den verwendeten Epoxidverbindungen Bestandteil eines Glycidol-Restes.

Um gleichzeitig die Thromboseresistenzeigenschaften zu verbessern und die freien Epoxidgruppen, die sich bei der Konservierung nicht mit dem Kollagen verbunden haben, zu schließen, d.h. umzusetzen, wird eine nachfolgende Behandlung des biologischen Materials mit Heparin und Acetylsalicylsäure durchgeführt.

Für die Konservierung von Herz-Kreislauf-Bioprothesen (Bioprothese = biologische Prothese) wird bevorzugt eine Mischung aus polymeren und nichtpolymeren Epoxidverbindungen verwendet.

Unter einer polymeren Epoxidverbindung wird im Sinne der Erfindung eine Epoxidverbindung verstanden, die wenigstens aus zwei sich wiederholenden, unmittelbar miteinander verbundenen Einheiten aufgebaut ist, mit denen Epoxygruppen-haltige Reste, wie zum Beispiel Glycidol bzw. 2,3-Epoxy-1-propanol, verbunden sind.

Bevorzugt weisen die polymeren Epoxidverbindungen einen Polymerisationsgrad von wenigstens zwei, weiter bevorzugt von drei bis 25, weiter bevorzugt von drei bis 15, insbesondere von vier bis neun, auf. Der Polymerisationsgrad bezieht sich dabei auf den die Epoxygruppen tragenden polymeren Anteil der Epoxidverbindung.

Anstelle einer polymeren Epoxidverbindung kann auch eine Epoxidverbindung mit zwei bis drei Epoxidgruppen, wobei zwischen wenigstens zwei Epoxidgruppen eine gerad- oder verzweigkettige Kohlenwasserstoffkette mit wenigstens vier Kohlenstoffatomen, beispielsweise eine Tetramethylengruppe, angeordnet ist, verwendet werden. Bevorzugt ist zwischen den wenigstens zwei Epoxidgruppe eine gerad- oder verzweigtkettige Kohlenwasserstoffkette mit wenigstens sechs, bevorzugt sechs, Kohlenstoffatomen, wie beispielsweise eine Hexamethylengruppe, angeordnet.

Unter einer nichtpolymeren Epoxidverbindung wird im Sinne der Erfindung eine Epoxidverbindung verstanden, die keine sich wiederholenden, unmittelbar miteinander verbundenen Einheiten aufweist, mit denen Epoxygruppen-haltige Reste, wie zum Beispiel Glycidol bzw. 2,3-Epoxy-1-propanol, verbunden sind.

Es kann vorteilhaft sein, die Zusammensetzung der Mischung in Abhängigkeit von der Art- und Gewebezugehörigkeit des biologischen Materials zu variieren. Dies steht im Zusammenhang mit der räumlichen Konfiguration und der Zusammensetzung der Kollagene der verschiedenen Gewebe sowie der unterschiedlichen Zugänglichkeit der reaktionsfähigen Aminosäuregruppen des Kollagens für die Konservierungsmittel, welche unterschiedliche Strukturformeln haben.

Außerdem hängt die Zusammensetzung der Mischung von der Aufgabe der zusätzlichen chemischen Modifikation ab: eine Sättigung des biologischen Materials mit einer vorgegebenen Anzahl von Epoxidgruppen, die für eine kovalente Immobilisierung von biologisch aktiven Stoffen erforderlich sind, ist möglich. So ist zum Beispiel bei einer Immobilisierung von Heparin eine große Anzahl von Epoxidgruppen erforderlich.

Hierzu wird das Gemisch bevorzugt wie folgt eingestellt:

40-80 Gew.-%, weiter bevorzugt 50-70 Gew.-%, wenigstens einer nichtpolymeren Epoxidverbindung mit zwei Epoxidgruppen;

5-20 Gew.-%, weiter bevorzugt 10-15 Gew.-%, wenigstens einer polymeren Epoxidverbindung mit zwei bis drei Epoxidgruppen und/oder wenigstens eine Epoxidverbindung mit zwei bis drei Epoxidgruppen, wobei zwischen wenigstens zwei Epoxidgruppen ein gerad- oder verzweigkettige Kohlenwasserstoffkette mit wenigstens vier Kohlenstoffatomen angeordnet ist,

15-45 Gew.-%, weiter bevorzugt 20-35 Gew.-%, wenigstens einer Epoxidverbindung mit wenigstens drei Epoxidgruppen;

wobei die Gesamtmenge 100 Gew.-% beträgt.
Die vorstehenden prozentualen Angaben der jeweiligen Epoxidverbindung in Gewichtsprozent beziehen sich jeweils auf die Gesamtmenge an eingesetzten Epoxidverbindungen.

Bei einer Modifikation mit hochmolekularen Polymerstoffen muß hingegen die Anzahl von reaktionsfähigen Gruppen gering sein, da eine Übersättigung des Gewebes der biologischen Prothese mit hochmolekularen Polymeren zu einer Verschlechterung der Elastizitäts- und Deformationseigenschaften führt. Dementsprechend ermöglicht der Einsatz von Epoxid-Mischungen unterschiedlicher Zusammensetzung eine Steuerung oder Beeinflussung der Stoffmenge, die auf dem biologischen Material immobilisiert wird.

Hierzu wird das Gemisch bevorzugt wie folgt eingestellt:

70-90 Gew.%. weiter bevorzugt 75-85 Gew.-%, wenigstens einer nichtpolymeren Epoxidverbindung mit zwei Epoxidgruppen,

5-20 Gew.-%, weiter bevorzugt 10-15 Gew.-%, wenigstens einer polymeren Epoxidverbindung mit zwei bis drei Epoxidgruppen und/oder wenigstens eine Epoxidverbindung mit zwei bis drei Epoxidgruppen, wobei zwischen wenigstens zwei Epoxidgruppen eine gerad- oder verzweigkettige Kohlenwasserstoffkette mit wenigstens vier Kohlenstoffatomen angeordnet ist,

bis zu 5 Gew.-%. weiter bevorzugt 1-2 Gew.-%, wenigstens einer Epoxidverbindung mit wenigstens drei Epoxidgruppen,

wobei die Gesamtmenge 100 Gew.-% beträgt.

Die vorstehenden prozentualen Angaben der jeweiligen Epoxidverbindung in Gewichtsprozent beziehen sich jeweils auf die Gesamtmenge an eingesetzten Epoxidverbindungen.

Bei den vorstehend angegebenen Gemischen muß in der Mischung eine nichtpolymere, bevorzugt niedrigmolekulare, Verbindung, mit zwei Reaktionsgruppen vorhanden sein. Beispielsweise können Alkandioldiglycidylether wie z.B. 1,4-Butandioldiglycidylether, Polyalkoholdiglycidylether wie z.B. Glycerindiglycidylether, Alkylenglykoldiglycidylether wie z.B. Ethylenglykoldiglycidylether oder Mischungen davon verwendet werden. Vorzugsweise wird Ethylenglykoldiglycidylether verwendet.

Diese Verbindungen gewährleisten eine intermolekulare Quervernetzung des Kollagens und die intramolekulare Vernetzung von Proteoglykanen und Eiweißen der Zellelemente, die die aktivsten antigenen Determinanten darstellen.

Die Konzentration an nichtpolymerer Epoxidverbindung mit zwei Epoxidgruppen (Diepoxidverbindung) in der Mischung beträgt bevorzugt 1-95 Gew.-%, weiter bevorzugt 20-90 Gew.-%. Sehr gute Ergebnisse wurden mit einer Konzentration von 40-80 Gew.-% erhalten.

Als zweite Komponente in dem Gemisch wird eine polymere Epoxidverbindung, die zwei bis drei Epoxidgruppen in der Strukturformel enthält, und/oder eine Epoxidverbindung mit zwei bis drei Epoxidgruppen, wobei zwischen wenigstens zwei Epoxidgruppen eine gerad- oder verzweigkettige Kohlenwasserstoffkette mit wenigstens vier Kohlenstoffatomen angeordnet ist, verwendet.

Beispielsweise können Polyalkylenglykoldiglycidylether wie z.B. Polyethylenglykoldiglycidylether, Polytetramethylenglykoldiglycidylether, Polypropylenglykoldiglycidylether oder Mischungen davon verwendet werden.

Anstelle oder zusätzlich zu den vorgenannten polymeren Epoxidverbindung können auch Alkandioldiglycidylether wie z.B. 1,6-Hexandioldiglycidylether und/oder höhere Dicarbonsäurediglycidylester verwendet werden.

Unter einem höheren Dicarbonsäurediglycidylester werden im Sinne der Erfindung mit höheren Dicarbonsäuren verestertes Glycidol (2,3-Epoxy-1-propanol) verstanden, d.h. Diglycidylester höherer Dicarbonsäuren. Als höhere Dicarbonsäuren werden solche bezeichnet, die mehr als 12 Kohlenstoffatome aufweisen.

Beispielsweise können als höhere Dicarbonsäurediglycidylester Denacol EX-1111 (Mischung aus zwei Säuren mit einem Molekulargewicht von 398 g/mol und 454 g/mol) oder Denacol EX-1112 (Mischung aus zwei Säuren mit gleichem Molekulargewicht von 450 g/mol, jedoch verschiedener Struktur) der Firma Nagase Company Ltd., Japan, verwendet werden.

Vorzugsweise wird Polyethylenglykoldiglycidylether verwendet. Vorzugsweise weist der Polyethylenglykoldiglycidylether einen Polymerisationsgrad von 3-12, weiter vorzugsweise von 4-9, auf.

Diese Komponente ergänzt die Vernetzung des Kollagens durch zwischenfibrilläre Verbindungen. Die Konzentration in der Mischung beträgt bevorzugt 1-95 Gew.-%, weiter bevorzugt 2-40 Gew.-%, noch weiter bevorzugt 5-20 Gew.-%.

Die dritte Komponente ist eine Epoxidverbindung mit einer Epoxidgruppenanzahl von wenigstens drei, beispielsweise vier, fünf oder mehr.

Bevorzugt werden dabei Polyalkoholpolyglycidylether wie z.B. Sorbitolpolyglycidylether, Glycerinpolyglycidylether, u.a., Polysaccharidpolyglycidylether oder Mischungen davon verwendet. Vorzugsweise wird Pentaerythrolpolyglycidylether verwendet.

Diese dritte Komponente sättigt das Gewebe mit einer optimalen Anzahl an freien Epoxidgruppen, an denen man Heparin ohne den Einsatz zusätzlicher Reagenzien immobilisieren kann. Die Konzentration der Komponente in der Mischung beträgt bevorzugt 1-95 Gew.-%, weiter bevorzugt 10-50 Gew.-%. Sehr gute Ergebnisse wurden mit einer Konzentration von 15-35 Gew.-% erhalten.

Die vorstehenden prozentualen Angaben der jeweiligen Epoxidverbindung in Gewichtsprozent beziehen sich jeweils auf die Gesamtmenge an eingesetzten Epoxidverbindungen.

Das erfindungsgemäße Verfahren ermöglicht eine Erhöhung der Dichte der Quervernetzung des Kollagens, was sich vorteilhaft auf die Festigkeitseigenschaften der biologischen Prothese auswirkt. Der Einsatz von Mischungen aus polymeren und nichtpolymeren Epoxiden verleiht dem biologischen Material eine höhere Resistenz gegen Kalzifikation.

Die Sättigung des Gewebes mit freien Epoxidgruppen ermöglicht die Realisierung einer kovalenten Immobilisierung von Heparin auf der so vorbehandelten biologischen Prothese.

Bezogen auf die funktionellen Gruppen des Heparin stellt die Epoxygruppe eine der reaktionsfähigsten Gruppen in den Polymeren, darunter auch in biologischen Materialien, dar. Die Bindung des Heparins über die Aminogruppe wirkt sich nicht nachteilig auf dessen antikoagulante Eigenschaften aus.

Das Umsetzen mit der Epoxygruppe ermöglicht die Realisierung einer Immobilisierung von Heparin unter "weichen" Bedingungen, die unerwünschte physikalisch-chemische Änderungen des biologischen Materials ausschließen. Das Umsetzen von Heparin mit den Epoxygruppen kann in einem breiten pH-Bereich des Mediums erfolgen (pH 2-11).

Bei der Modifikation des biologischen Gewebes wird vorzugsweise ein pH-Wert 5-8, beispielsweise unter Verwendung von Phosphatpuffern, Phosphat-Citrat-Puffern und/oder Acetylsalicylsäure, eingestellt.

Eine solche Herangehensweise ermöglicht es einerseits, eine Verbesserung der thromboseresistenten Eigenschaften zu erreichen, und andererseits die freien Epoxygruppen zu neutralisieren, die als Ergebnis des Maskier-Effektes stets im biologischen Material vorhanden sind.

Die Einführung einer dritten Komponente, d.h. einer Verbindung mit einer Epoxygruppenanzahl von wenigstens 3, in das Epoxidkonservierungsmittel ermöglicht eine Erhöhung der Menge an immobilisiertem Heparin. Das kovalent gebundene, d.h. immobilisierte Heparin gelangt nicht in den Blutstrom, hat aber durch die Bindung und Sorption an die Eiweißschichten und die Glättung der Oberfläche der biologischen Prothese einen antithrombotischen Effekt.

Die Verwendung von Acetylsalicylsäure für die Einstellung eines sauren pH-Wertes bei der Immobilisierung von Heparin erhöht überraschenderweise weiter die Thromboseresistenz der Oberfläche.

Zur Herstellung einer erfindungsgemäßen konservierten biologischen Prothese für die Herz-Kreislauf-Chirurgie, wie beispielsweise von biologischen Herzklappenprothesen, können z.B. Aortenkomplexe des Schweins oder Perikarde des Rinds verwendet werden. Im Falle einer Arteriosklerose können beispielsweise geschädigte Arterien durch die innere Brustschlagader oder die Kopfschlagader des Rinds ersetzt werden.

Für den orthotopischen Ersatz von Herzklappen können allogene oder heterologe Mitralklappen oder Trikuspidalklappen verwendet werden. Für die intrakardiale Plastik und die Angioplastik können Perikarde des Rinds, die allogene harte Hirnhaut oder körpereigene Perikarde verwendet werden. Klappen, membranenähnliche Gewebe und Gefäße, beispielsweise Arterien, Venen, Arteriensegemente, Venensegemente, usw., können von beliebigen biologischen Gattungen von Säugetieren, beispielsweise Rind, Schwein, Schaf, Mensch, usw., entnommen werden, sofern diese sich ihren anatomischen Merkmalen nach für einen Ersatz des einen oder anderen Elementes des Herz-Kreislauf-Systems eignen.

Die nachfolgend aufgeführten erfindungsgemäßen Beispiele 1 bis 3 dienen lediglich der weiteren Veranschaulichung der Erfindung und schränken in keiner Weise den Schutzumfang ein.

In den erfindungsgemäßen Beispielen wurde zur Konservierung der jeweils verwendeten biologischen Prothese eine Mischung aus Ethylenglykoldiglycidylether (DEE), Pentaerythrolpolyglycidylether und Polyethylenglykoldiglycidylether in den jeweils angegebenen Mengenverhältnissen eingesetzt.

Die Beispiele wurden dabei sämtlichst bei Umgebungstemperatur durchgeführt, d.h. bei etwa 20°C bis etwa 25°C. Es können auch höhere Temperaturen verwendet werden, jedoch nicht mehr als 37°C. Bei sämtlichen Inkubationsschritten wurden die Lösungen nicht gerührt. Selbstverständlich können die Lösungen aber auch gerührt werden.

## Erfindungsgemäßes Beispiel 1

Aortenklappensegel des Schweins (15 g feuchtes Gewebe) wurden mit 0,9 Gew.-%iger NaCl-Lösung gespült und in 200 ml einer erfindungsgemäßen Konservierungslösung gegeben, die aus 50 mM Phosphatpuffer pH 7,4 hergestellt ist und 6 g Ethylenglykoldiglycidylether, 1 g Polyethylenglykoldiglycidylether (n=5) und 3 g Pentaerythrolpolyglycidylether (Anzahl der Glycidylether-Einheiten pro Molekül: 4) enthält.

Nach 48 h wurde die Konservierungslösung gegen eine identische, aber frisch hergestellte Lösung ausgetauscht. Nach 12 Tagen wurden die Klappensegel mit steriler 0,9 Gew.-%iger NaCl-Lösung gespült und in einer Heparinlösung (100 IU/ml, IU: Internationale Einheit) pH 5,0, wobei der pH-Wert durch Zugabe von wäßriger Acetylsalicylsäurelösung eingestellt wurde, für eine Zeitdauer von 3 Stunden bei einer Temperatur von 20°C inkubiert.

Danach wurde fünfmal mit einem Überschuß an 0,9 Gew.-%iger NaCl-Lösung gespült und die behandelten Klappensegel in 5 Gew.-%ige Ethylenglykoldiglycidyletherlösung gegeben, wo sie bis zur weiteren Verwendung aufbewahrt wurden.

## Herstellung der Heparinlösung

Die Verhältnis von Heparin : Acetylsalicylsäure in der Heparinlösung beträgt, bezogen auf das Gewicht, etwa 1,3-16 : 1. Das genaue Verhältnis von Heparin : Acetylsalicylsäure hängt von der jeweiligen Aktivität (in IU/Gewicht) des verwendeten Heparins ab.

Beispielsweise kann eine für die Erfindung geeignete Heparinlösung durch Zugabe von etwa 7-10 mg/ml Acetylsalicylsäure zu einer Heparinlösung mit etwa 75-100 IU/ml hergestellt werden. Dabei wird Acetylsalicylsäure zu der Heparinlösung zugegeben, bis der pH-Wert der Lösung etwa 5 bis 6 beträgt. Die Konzentration der Heparinlösung beträgt wenigstens 75 IU/ml, bevorzugter 100 IU/ml. Selbstverständlich können auch Lösungen mit höheren Heparinkonzentrationen verwendet werden.

## Vergleichsbeispiele 1

Als Vergleichproben wurden Aortenklappensegel des Schweins verwendet, die mit 0,625 Gew.-% Glutaraldehyd (GA) in 50 mM Phosphatpuffer pH 7,4 bzw. mit 5 Gew.-% Ethylenglykoldiglycidylether (DEE) und 100 IU/ml Heparin in 50 mM Phosphatpuffer pH 7,4 (DEE + Heparin)(siehe RU 2,008,767) behandelt wurden.

a) Behandlung von Proben mit Glutaraldehyd

Die Vergleichsproben wurden für 28 Tage in 0,625 Gew.-% Glutaraldehyd (GA), 50 mM Phosphatpuffer pH 7,4 bei Zimmertemperatur (20°C bis 25°C) inkubiert. Die GA-Lösung wurde viermal, d.h. nach dem 1., 3., 7. und nach dem 21. Tag gewechselt. Vor der weiteren Verwendung (Analyse oder Implantation) wurde die Konservierungslösung entfernt. Nachfolgend wurde die Vergleichsprobe bei Zimmertemperatur für eine Stunde ohne Rühren in 0,9%iger Kochsalzlösung gewaschen. Die Kochsalzlösung wurde dabei jeweils nach 20 Minuten gewechselt (insgesamt drei Waschschritte).

b) Behandlung von Proben mit DEE und Heparin

Die Vergleichsprobe wurde für 21 Tage in 5 Gew.-% Ethylenglykoldiglycidylether (DEE), 50 mM Phosphatpuffer pH 7,4 bei Zimmertemperatur (20°C-25°C) inkubiert. Die DEE-Lösung wurde nach drei Tagen ohne Waschschritt gewechselt. Anschließend wurde die Konservierungslösung entfernt. Nachfolgend wurde die Vergleichsprobe bei Zimmertemperatur für eine Stunde ohne Rühren in 0,9%iger Kochsalzlösung gewaschen. Die Kochsalzlösung wurde dabei jeweils nach 20 Minuten gewechselt (insgesamt drei Waschschritte). Die Heparin-Behandlung erfolgte mit 100 IU/ml bei 37°C für 8-16 Stunden. Das ungebundene Heparin wurde durch Waschen mit 0,9 Gew.-%iger Kochsalzlösung für eine Stunde bei Zimmertemperatur (20-25°C) entfernt. Während des Waschvorgangs wurde die Kochsalzlösung nicht gerührt und nicht gewechselt. Die Vergleichsprobe wurde in 2-5 Gew.-%iger DEE-Lösung aufbewahrt. Vor der weiteren Verwendung (Analyse oder Implantation) wurde die Konservierungslösung entfernt. Nachfolgend wurde die Vergleichsprobe bei Zimmertemperatur für eine Stunde ohne Rühren in 0,9%iger Kochsalzlösung gewaschen. Die Kochsalzlösung wurde dabei jeweils nach 20 Minuten gewechselt (insgesamt drei Waschschritte).

Vergleich von erfindungsgemäßen Beispielen und Vergleichsbeispielen

Die Verbesserung der Eigenschaften von nach dem erfindungsgemäßen Verfahren konservierten biologischen Prothesen (erfindungsgemäße Beispiele 1 bis 3) wird durch Vergleich von mit herkömmlichen Konservierungsverfahren konservierten biologischen Prothesen (Vergleichsbeispiele 1 bis 3) veranschaulicht. Dabei wurden die Dichte der Quervernetzung von Aminosäuren in den jeweils verwendeten biologischen Prothesen, die Elastizitäts- und Deformationseigenschaften, der Kalzifikationsgrad sowie die Menge an immobilisiertem Heparin bestimmt und einander gegenübergestellt.

Die vorgenannten Parameter wurden dabei wie folgt bestimmt.

Bestimmung der Quervernetzung

Die Dichte der Quervernetzung wurde nach der Verringerung der Anzahl freier Aminosäurereste im biologischen Material bewertet, wobei diese durch eine Aminosäureanalysenmethode bestimmt wurden.

Für die Aminosäureanalyse wurden jeweils fünf Proben genommen und mit destilliertem Wasser gewaschen, das in einer Stunde zweimal erneuert wurde. Nachfolgend wurden die Proben für drei Stunden lyophilisiert (Temperatur der Probe: -55°C bis +60°C). 1,5 bis 2 mg trockenes Gewebe wurden in 0,15 ml bis 2 ml 6 N HCl aufgenommen und in abgedichten Vakuumampullen für 24 Stunden bei 110°C inkubiert. Danach wurde die Säure verdampft, und der Rückstand wurde in 2,5 ml Lithiumcitratpuffer verdünnt und zentrifugiert. Der Überstand wurde einer Aminosäureanalyse in einem Aminosäureanalysator (CL 5001 BIOTRONIC, Deutschland) mit rechnergestützten Datenauswertung (CR-3A, SHIMADZU Integrator, Japan) unterworfen.

Bestimmung der Elastizitäts- und Deformationseigenschaften

Die Proben wurden mit einer speziell geformten Klinge in Dumbbell-Form (Hantelprüfkörper) ausgeschnitten. Diese Klinge weist eine Standardform und - größe sowie geschärfte Kanten auf. Unter Verwendung dieser Klinge werden Proben mit standardisierter Größe aus dem biologischen Material herausgeschnitten. Es wurden jeweils zehn Proben untersucht.

Die Elastizitäts- und Deformationseigenschaften wurden auf der Zugfestigkeits-Prüfmaschine "Instron-1122" (Hersteller: INSTRON, England) bestimmt.

Sämtliche untersuchten Materialien wurden zum Untersuchen der Bruch/Reißfestigkeit bei einer konstanten Geschwindigkeit (50 mm/min) belastet.

Die durchschnittliche Dicke h der Proben (mm) wurde unter Verwendung eines Mikrometerokulars bestimmt.

Die Daten wurden wie folgt berechnet:

a) Maximale Zugfestigkeit (Zugspannung) $\sigma$ (kg/cm$^2$)

$\sigma = P_{max}/S$, wobei $P_{max}$ die Bruchlast (kg) und S die Querschnittsfläche der Probe ist (cm$^2$)

S= h x $b_0$, wobei h (cm) die Dicke der Probe und $b_0$ (cm) die Breite der Probe ist. Vorliegend wurde die Breite der Probe auf 0,25 cm unter Verwendung der oben erwähnten Klinge eingestellt.

b) Maximale Dehnung $\varepsilon_{max}$ (%)

$$\varepsilon_{max}= (\Delta l_{max}/l_0) \times 100,$$

$\Delta l_{max} = l_{max}-l_0$, wobei "$l_{max}$" die Endlänge und "$l_0$" die Anfangslänge der Probe ist.

Die Proben wiesen vorliegend eine Anfangslänge von 11 mm auf.

Bestimmung der Kalzifikation

Die Resistenz gegenüber Kalzifikation wurde durch eine subkutane Implantation konservierter biologischer Prothesen unter die Haut von 3-wöchigen männlichen Ratten untersucht (Durchmesser der Proben: 7 mm).

Drei Proben, jeweils eine der unter Verwendung der GA, DEE + Heparin und der erfindungsgemäßen Konservierungslösung konservierten biologischen Prothesen, wurden jeweils in eine von männlichen Vistar-Ratten (n=33), Gewicht 48,64 ± 3,5 g, unter Ethemarkose implantiert. Die Implantate wurden nach einem Zeitraum von 30, 60 und 90 Tagen jeweils 11 Tieren entnommen.

Nach der Entnahme der Proben wurden diese von dem umgebenden Gewebe gesäubert, mit 0,9 Gew.-%iger NaCl-Lösung gewaschen und bei 56°C für einen Tag getrocknet. Nachfolgend wurde jede der Proben in konzentrierter Chlorsäure hydrolysiert. Die Proben wurden dann auf Calcium mittels Atomabsorptions-Spektroskopie quantitativ untersucht.

Bestimmung der Menge an immobilisiertem Heparin

Die Menge an immobilisiertem Heparin wurde mittels Elementaranalyse nach der Differenz des Gehalts an Schwefel in nichtmodifizierten (Kontrollprobe) und modifizierten Probestücken (mod. Probe) bestimmt.

Das Verfahren beruht auf der Bestimmung des Unterschieds der Schwefelkonzentration in Versuchsproben (mod. Probe) und Kontrollproben. Der Schwefelgehalt in Heparin ist groß und in Kollagen gering. Ein Anstieg des Schwefelgehalts nach Modifizierung der biologischen Prothese ermöglicht mithin eine Berechnung der Menge des an der biologischen Prothese immobilisierten Heparins.

$$\Delta[S]\text{Heparin} = [S] \text{ mod. Probe} - [S] \text{ Kontrollprobe,}$$

wobei [S] die Schwefelkonzentration in [$\mu$g/g] Trockengewicht ist.

Die Berechnung des Heparingehalts in der Probe erfolgt dabei nach folgender Formel:

$Y = \Delta[S]/S_1$, wobei Y der Heparingehalt in dem biologischen Material (biologische Prothese) in [mg/g] ist, $S_1$ ist der Schwefelgehalt in [$\mu$g] in 1 mg Heparin.

Zur Bestimmung der Konzentration des Schwefelgehalts kann grundsätzlich jedes quantitative Bestimmungsverfahren verwendet werden.

Vorliegend erfolgte die Bestimmung wie folgt:

Das Verfahren zur Bestimmung der Konzentration des Schwefels - der im Bereich von 0,2 bis 100 % in organischen Proben enthalten ist - basiert auf der Titration eines wässrig-organischen Mediums nach Verbrennung der Probe in einem sauerstoffhaltigen Kolben.

Das Probenmaterial (wenigstens 20 g) wird zunächst bis zum Erhalt einer dünnbreiigen Konsistenz mit einer Schere zerkleinert. Jede Probe wird mit 10 ml destilliertem Wasser übergossen. Die Probe wird auf -55°C eingefroren und unter schrittweiser Erhöhung der Temperatur auf +60°C bis zur Trockne lyophilisiert. Das so erhaltene getrocknete Probenmaterial wird im Achatmörser bis zum Erhalt eines feinen Pulvers verrieben.

5 mg des Probenmaterial werden auf einer Analysenwaage mit einer Teilung von 0,0001 g abgewogen. Die Einwaage wird in einem Kolben, der mit gasförmigen Sauerstoff befüllt ist und 10 ml 6% $H_2O_2$-Lösung enthält, verbrannt. Nach der Verbrennung werden die Verbrennungsprodukte mit 5 ml Wasser abgespült und abgekühlt. Zu dieser Lösung werden 0,25 ml 2 N HCl, 25 ml Aceton und 2 Tropfen Indikator (0,2 % wässrige Chlorphosphonazo-III-Lösung (Bis-(4-chlor-2-phosphonobenzolazo)-2,7-chromotropsäure, Fluka Chemie AG, CH-9471 Buchs, Schweiz) gegeben. Die Titration erfolgt mit 0,02 N Ba(NO$_3$)$_2$-Lösung bis zum Übergang von einer Violett-Rosa-Färbung in eine hellblaue Färbung. Zur Kontrolle wird ein Leerversuch unter Analysebedingungen einschließlich Verbrennung und Titration durchgeführt.

Der Schwefelgehalt X wird wie folgt berechnet:

$$X = \frac{(V-V_0) \times K}{A} \times 1000,$$

wobei:

- V das für die Titration verbrauchte Volumen an 0,02 N Ba(NO$_3$)$_2$-Lösung in [ml] ist,
- $V_0$ das für die Titration bei dem Leerversuch verbrauchte Volumen an 0,02 N Ba(NO$_3$)$_2$-Lösung in [ml] ist,
- K der Umrechnungsfaktor, der den Titer der Ba(NO$_3$)$_2$-Lösung für das Schwefeläquivalent in [mg/ml] wiedergibt,
- A das Gewicht der Probe in [mg] ist.

Die Menge an immobilisiertem Heparin kann selbstverständlich auch auf anderem Wege bestimmt werden. Beispielsweise ist eine Quantifizierung von immobilisiertem Heparin unter Verwendung von Toluidinblau, das an immobilisiertes Heparin bindet, möglich.

Beide Verfahren führen zu einem gleichen Ergebnis.

Ergebnis Beispiel 1/Vergleichsbeispiele 1

Tabelle 1

| Tabelle 1 zeigt den relativen Gehalt an freien Aminosäureresten (bezogen auf 1000 Aminosäurereste) in Aortenklappensegeln des Schweins. | | | | |
|---|---|---|---|---|
| Aminosäure | natives Gewebe | GA | DEE + Heparin | erfindungsgemäßes Bsp. |
| THR | 27,3±0,2 | 27,6±0.6 | 24,5±0,3 | 23,2±0,5 |
| SER | 45,3±0,4 | 46,7±1,1 | 41,6±0,4 | 39,6±0,4 |
| GLU | 97,7±0,0 | 103,0±0,5 | 89,1±0,7 | 88,2±1,2 |
| OHPRO | 110,4±1,2 | 117,6±1,2 | 115,6±3,9 | 110,8±1,8 |
| PRO | 25,7±0,5 | 28,6±0,4 | 65,6±0,6 | 64,8±1,7 |
| GLY | 238,2±1,2 | 252,1±4,1 | 261,1±2,4 | 272,7±3,2 |
| ALA | 124,3±0,9 | 127,7±0,8 | 122,3±0,5 | 132,2±3,1 |
| VAL | 44,3±0,8 | 41,9±1,2 | 42,0±1,7 | 37,1±1,4 |
| **MET** | **10,7±0,3** | **10,6±0,2** | **-** | **-** |
| ILE | 19,9±0,3 | 20,2±0,3 | 16,0±0,3 | 16,7±0,5 |
| LEU | 42,4±0,3 | 42,6±0,6 | 36,2±0,3 | 36,1±0,5 |
| **TYR** | **10,4±0,3** | **9,4±0,2** | **8,7±0,2** | **1,9±0,5** |
| PHE | 21,9±0,4 | 20,9±0,4 | 22,6±0,7 | 19,7±1,4 |
| HIS | 12,9±0,2 | 15,8±0,3 | 35,6±0,4 | 39,2±0,6 |
| **OHLYS** | **11,0±0,2** | **1,4±0,2** | **2,3±0,2** | **-** |
| **LYS** | **34,0±0,3** | **3,3±0,2** | **7,4±0,2** | **-** |
| ASP | 66,5±0,5 | 70,0±0,5 | 61,2±0,5 | 60,1±0,9 |
| ARG | 57,1±0,8 | 63,1±1,2 | 47,8±0,9 | 56,3±1,3 |

Es ist bekannt, daß Epoxidverbindungen mit Methionin, Tyrosin, Lysin und Hydroxylysin des biologischen Materials reagieren, wohingegen Glutaraldehyd nur mit den beiden letzten Aminosäuren reagiert. Die vorgelegten Ergebnisse bestätigen dies.

Hierbei ist beim Einsatz des erfindungsgemäßen Konservierungsmittels die Dichte der Quervernetzung größer, als beim Einsatz des Einzelstoffes - Ethylenglykoldiglycidylether. Dies geht insbesondere aus einer Abnahme des relativen Gehalts der Aminosäuren Methionin, Tyrosin, Lysin und Hydroxylysin hervor.

Tabelle 2

| Tabelle 2 zeigt die physikalisch-mechanischen Parameter von mit verschiedenen Verfahren konservierten Aortenklappensegeln des Schweins. | | | |
|---|---|---|---|
| Konservierungsmittel | $\sigma$ [kg/cm$^2$] | $\varepsilon$ [%] | h [cm] |
| Glutaraldehyd (GA) | 59,2±4,6 | 38,7±1,9 | 0,059±0,003 |
| DEE + Heparin | 69,9±5,9 | 38,7±1,8 | 0,046±0,002 |
| erfindungsgem. Bsp. | 93,5±8,0 | 35,9±1,6 | 0,041±0,002 |
| Anmerkung: $\sigma$: Bruchspannung bei Zugbeanspruchung, $\varepsilon$: relative Dehnung, h: Gewebedicke | | | |

Die gemäß dem erfindungsgemäßen Beispiel hergestellte Probe weist bei geringerer Dicke ein besseres Zugfestigkeitsverhalten (größere Bruchspannung) auf.

Tabelle 3

| Tabelle 3 zeigt die Calciummenge (mg/g trockenes Gewebe) in unter die Haut von Ratten implantierten Klappensegelstücken zu verschiedenen Zeiträumen nach der Implantation. | | | |
|---|---|---|---|
| Implantationszeitraum | GA | DEE + Heparin | erfindungsgemäßes Bsp. |
| Ohne Implantation | 2,25±0,10 | 2,18±0,08 | 2,23±0,11 |
| 30 Tage | 125,6±10,2 | 2,5±0,07 | 2,15±0,08 |
| 60 Tage | 211,7±12,4 | 2,8±0,10 | 2,3±0,07 |
| 90 Tage | 265,4±21,3 | 2,5±0,09 | 2,7±0,11 |

Die gemäß dem erfindungsgemäßen Beispiel hergestellte Probe weist einen äußerst geringen Calcifikationsgrad auf.

Tabelle 4

| Tabelle 4 zeigt die Menge an immobilisiertem Heparin. | |
|---|---|
| DEE + Heparin | erfindungsgemäßes Beispiel |
| 530±20 µg/g trockenes Gewebe | 2340±120 µg/g trockenes Gewebe |

Die gemäß dem erfindungsgemäßen Beispiel hergestellte Probe weist einen sehr hohen Gehalt an immobilisiertem Heparin auf.

Erfindungsgemäßes Beispiel 2

Segmente der inneren Brustschlagader des Rinds (25 g feuchtes Gewebe) wurden mit 0,9 Gew.-%iger NaCl-Lösung gespült und in 200 ml einer Konservierungslösung, die aus 50 mM HEPES-Puffer pH 7,4 hergestellt ist und 8 g Ethylenglykoldiglycidylether, 0,5 g Polyethylenglykoldiglycidylether (n=6) und 1,5 g Pentaerythrolpolyglycidylether (Anzahl der Glycidylether- Einheiten pro Molekül: 4) enthält, gegeben.

Nach 48 h wurde die Konservierungslösung gegen eine identische, aber frisch hergestellte Lösung ausgetauscht. Nach 12 Tagen wurden die Segmente mit steriler 0,9 Gew.-%iger NaCl-Lösung gespült und in einer Heparinlösung (100 IU/ml) pH 5,0, wobei der pH-Wert durch Zugabe von wäßriger Acetylsalicylsäurelösung (Herstellung siehe erfindungsgemäßes Beispiel 1) eingestellt wurde, für eine Zeitdauer von 4 Stunden bei einer Temperatur von 20 °C inkubiert. Die Herstellung der Heparinlösung erfolgte wie in Beispiel 1 beschrieben.

Danach wurden die Segmente dreimal mit einem Überschuß an 0,9 Gew.-%iger NaCl-Lösung gespült und in eine 5 Gew.-%ige Ethylenglykoldiglycidyletherlösung geben, wo sie bis zur weiteren Verwendung aufbewahrt wurden.

Vergleichsbeispiele 2

Als Vergleichproben wurden Segmente der inneren Brustschlagader des Rinds verwendet, die mit 0,625 Gew.-%ige Glutaraldehyd in 50 mM Phosphatpuffer pH 7,4 (GA) bzw. mit 5 Gew.-% Ethylenglykoldiglycidylether und 100 IU/ml Heparin in 50 mM Phosphatpuffer pH-Wert 7,4 (DEE + Heparin) (siehe RU 2,008,767) behandelt wurden. Die Herstellung der Vergleichsproben erfolgte entsprechend der bei den Vergleichsbeispielen 1 angegebenen Beschreibung.

Ergebnis Beispiel 2/Vergleichsbeispiele 2

Die Bestimmung der Dichte der Quervemetzung, des Kalzifikationsgrades sowie der Menge an immobilisiertem Heparin erfolgte wie in oben bzgl. Beispiel 1 beschrieben. Die in Beispiel 1 und Vergleichsbeispielen 1 erhaltenen Ergebnisse werden durch vorliegend erhaltenen Ergebnisse bestätigt.

Die Beständigkeit gegenüber Thrombosebildung wurde nach der Implantation von Gefäßsegmenten, die gemäß dem erfindungsgemäßen Verfahren (erfindungsgem. Beispiel) bzw. zu Vergleichszwecken unter Verwendung von Glutaraldehyd (GA) bzw. Ethylenglykoldiglycidylether und Heparin (DEE + Heparin) konserviert wurden, in die Karotis-Arterie von Hunden bewertet.

Die Gefäßsegmente wiesen dabei einen Durchmesser von etwa 3 mm bis 3,5 mm und eine Länge von etwa 5 bis 7 cm auf und wurden in die Halsschlagader (Karotis) von 24 nicht reinrassigen Hunden, die ein Gewicht von etwa 10 bis 15 kg aufwiesen, implantiert.

Die Hunde wurden zuvor durch intravenöse Gabe von Natrium-Thiopental anästhesiert und mechanisch ventiliert. Den Hunden wurde ein 5 bis 7 cm langes Karotis-Arteriensegment entfernt und die Bioprothese an dieser Stelle unter Verwendung von 6-0 Polypropylen Nahtmaterial implantiert.

Acht Tiere erhielten eine "GA"-Bioprothese in die rechte Karotis-Arterie und eine "DEE + Heparin"-Bioprothese in die linke Karotis-Arterie.; acht weitere Tiere erhielten eine "DEE + Heparin"-Bioprothese in die rechte Karotis-Arterie und eine erfindungsgemäße Bioprothese in die linke Karotis-Arterie; acht weitere Tiere erhielten eine erfindungsgemäße Bioprothese in die rechte Karotis-Arterie und eine "GA"-Prothese in die linke Karotis-Bioprothese. Der Durchfluß in den Prothesen wurde vor dem Wundverschluß mittels Pulsationspalpation untersucht. Die Durchgängigkeit der Bioprothesen wurde mittels Angiographie und Ultraschallverfahren (Doppler-Echographie, Duplex-Scanning) bestimmt.

Die Auswertung der erhaltenen Daten erfolgte mittels der Aktuaranalyse. Die Aktuaranalyse ist ein standardisiertes statistisches Verfahren, das auf der Wahrscheinlichkeit von analysierten Komplikationen, vorliegend der Thrombose, beruht, wobei bereits eingetretene Ereignisse berücksichtigt werden. Die Auswertung der Ergebnisse erfolgte mit dem Programm STATISTICA für Windows (StatSoft, Inc., 1995).

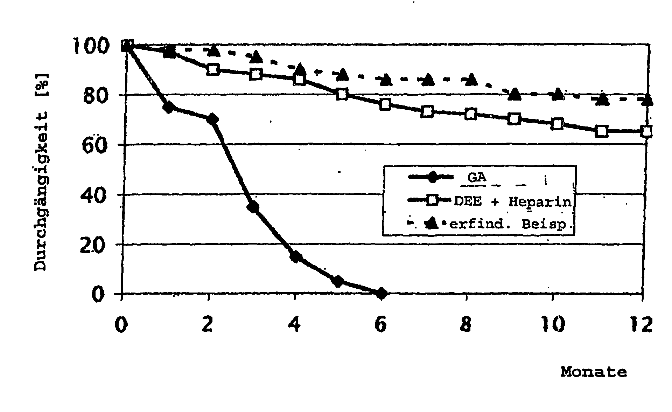

Die Aktuarwerte der Durchgängigkeit in [%] von in die Halsschlagader (Karotis) der Hunde implantierten biologischen Prothesen wurden bestimmt und sind in Tabelle 5 aufgeführt. Die in Tabelle 5 angegebenen Werte sind in Abbildung 1 graphisch veranschaulicht.

Tabelle 5

| Tabelle 5 zeigt die Aktuarwerte der Durchgängigkeit von implantierten Bioprothesen in [%]. | | | |
|---|---|---|---|
| Zeitraum in Monaten | GA | DEE + Heparin | erfindungsgem. Beispiel |
| 0 | 100 % | 100 % | 100% |
| 1 | 75 % | 97 % | 98 % |
| 2 | 70 % | 90 % | 98 % |
| 3 | 35% | 88% | 95% |
| 4 | 15% | 86% | 90% |
| 5 | 5% | 80% | 88% |
| 6 | 0% | 76% | 86% |
| 7 | | 73 % | 86 % |

Tabelle 5 (fortgesetzt)

| Zeitraum in Monaten | GA | DEE + Heparin | erfindungsgem. Beispiel |
|---|---|---|---|
| 8 | | 72 % | 86 % |
| 9 | | 70% | 80 % |
| 10 | | 68 % | 80 % |
| 11 | | 65% | 78% |
| 12 | | 65% | 78% |

Tabelle 6

| Tabelle 6 zeigt den relativen Gehalt an freien Methionin-, Tyrosin-, Lysin- und Hydroxylysinresten (bezogen auf 1000 Aminosäurereste) in Proben der inneren Brustschlagader des Rinds. | | | | |
|---|---|---|---|---|
| Aminosäure | natives Gewebe | GA | DEE + Heparin | erfindungsgem. Bsp. |
| MET | 7,5±0,5 | 7,3±0,4 | 6,3±0,2 | - |
| TYR | 10,0±0,6 | 11,0±0,4 | - | 3,4±0,5 |
| OHLYS | 7,0±0,5 | 1,0±0,2 | 0,9±0,2 | - |
| LYS | 24,1 ±1,9 | 3,5±0,5 | - | - |

Tabelle 7

| Tabelle 7 zeigt die Menge an immobilisiertem Heparin. | |
|---|---|
| DEE + Heparin | erfindungsgemäßes Beispiel |
| 190±10 μg/g trockenes Gewebe | 1005±90 μg/g trockenes Gewebe |

Tabelle 8

| Tabelle 8 zeigt die Calciummenge (mg/g trockenes Gewebe) in unter die Haut von Ratten implantierten Proben der inneren Brustschlagader des Rinds zu verschiedenen Zeiträumen nach der Implantation. | | | |
|---|---|---|---|
| Implantationszeitraum | GA | DEE + Heparin | erfindungsgem. Bsp. |
| Ohne Implantation | 1,39±0,11 | 1,5±0,03 | 1,42±0,09 |
| 30 Tage | 52,6±4.1 | 1,35±0,04 | 1,5±0,10 |
| 60 Tage | 74,1±9.3 | 1,8±0,07 | 1,9±0,07 |
| 90 Tage | 92,0±10,4 | 1.5±0.04 | 1.5±0,01 |

Erfindungsgemäßes Beispiel 3

Perikard des Rinds (30 g feuchtes Gewebe) wurde mechanisch vom umgebenden Gewebe gereinigt, mit 0,9 Gew.-%iger NaCl-Lösung gespült und in 200 ml einer Konservierungslösung, die aus 50 mM HEPES-Puffer pH 7,4 hergestellt ist und 4,5 g Ethylenglykoldiglycidylether, 1,5 g Polyethylenglykoldiglycidylether (n=4) und 4 g Pentaerythrolpolyglycidylether (Anzahl der Glycidylether- Einheiten pro Molekül: 4) enthält, inkubiert.

Nach 48 h wurde die Lösung gegen eine identische, aber frisch hergestellte Lösung ausgetauscht. Nach 12 Tagen wurde das Perikard mit steriler 0,9 Gew.-%iger NaCl-Lösung gespült und in einer Heparinlösung (100 IU/ml) pH 6,0, wobei der pH-Wert durch Zugabe von wäßriger Acetylsalicylsäurelösung (Herstellung siehe erfindungsgemäßes Beispiel 1) eingestellt wurde, für einen Zeitraum von 4 Stunden bei einer Temperatur von 20°C inkubiert.

Danach wurden das Perikard dreimal mit einem Überschuß an 0,9 Gew.-%iger NaCl-Lösung gespült und in eine 5 Gew.-%ige Ethylenglykoldiglycidyletherlösung geben, wo es bis zur weiteren Verwendung aufbewahrt wurden.

Vergleichsbeispiele 3

Als Vergleichproben wurden Perikardstücke des Rinds verwendet, die mit 0,625 Gew.-% Glutaraldehyd in 50 mM Phosphatpuffer pH 7,4 (GA) bzw. mit 5 Gew.-% Ethylenglykoldiglycidylether (DEE) und 100 IU/ml Heparin in 50 mM Phosphatpuffer pH-Wert 7,4 (DEE + Heparin)(siehe RU 2,008,767) behandelt wurden. Die Herstellung der Vergleichsproben erfolgte entsprechend der bei den Vergleichsbeispielen 1 angegebenen Beschreibung.
Die Bestimmung der Dichte der Quervernetzung, der Elastizitäts- und Deformationseigenschaften sowie der Menge an immobilisiertem Heparin erfolgte wie in Beispiel 1 beschrieben. Die in Beispiel 1 und Vergleichsbeispielen 1 erhaltenen Ergebnisse werden durch vorliegend erhaltenen Ergebnisse bestätigt.

Ergebnis erfindungsgemäßes Beispiel 3/Vergleichsbeispiele 3

Tabelle 9

| Tabelle 9 zeigt den relativen Gehalt an freien Methionin-, Tyrosin-, Lysin-, Hydroxylysin- und Argininresten (bezogen auf 1000 Aminosäureresten) in Perikarden des Rinds. | | | | |
|---|---|---|---|---|
| Aminosäure | natives Gewebe | GA | DEE + Heparin | erfindungsgem. Bsp. |
| MET | 8,8±0,5 | 8,7±0,5 | 4,4±0,3 | - |
| TYR | 8,2±0,2 | 8,0±0,4 | - | 5,0±0,3 |
| OHLYS | 9,9±0,4 | 1,1±0,1 | 0,7±0,2 | 1,5±0,1 |
| LYS | 32,0±1,9 | 2,8±0,1 | - | 1,4±0,3 |
| ARG | 55,6±0,8 | 58,6±0,5 | 50,9±0.8 | 12,7±0,3 |

Tabelle 10

| Tabelle 10 zeigt die Menge an immobilisiertem Heparin. | |
|---|---|
| DEE + Heparin | erfindungsgemäßes Bsp. |
| 760±1 0 µg/g trockenes Gewebe | 2640±85 µg/g trockenes Gewebe |

Tabelle 11

| Tabelle 11 zeigt die physikalisch-mechanischen Parameter von mit verschiedenen Verfahren konservierten Perikardstücken des Rinds. | | | |
|---|---|---|---|
| Konservierungsmittel | $\sigma$ [kg/cm$^2$] | $\varepsilon$ [%] | h [cm] |
| Glutaraldehyd | 119.8±6,2 | 48,5±5,8 | 0,046±0,003 |
| DEE + Heparin | 123,3±11,4 | 58,4±3,5 | 0,044±0,004 |
| erfindungsgem. Bsp. | 125,5±12,3 | 57,0±3,6 | 0,045±0,005 |
| Anmerkung: $\sigma$: Bruchspannung bei Zugbeanspruchung, $\varepsilon$: relative Dehnung, h: Gewebedicke | | | |

**Patentansprüche**

1. Verfahren zur Konservierung von biologischen Prothesen,
   **dadurch gekennzeichnet,**
   **daß** das Verfahren die Schritte umfaßt:

   (a) Behandeln von biologischen Prothesen mit einer Lösung, die ein Gemisch von Epoxidverbindungen, die wenigstens teilweise verschiedene Längen aufweisen, enthält;
   (b) Behandeln der gemäß Schritt (a) behandelten biologischen Prothese mit einer Antithrombotikum-haltigen Lösung, wobei das Antithrombotikum eine Mischung aus Heparin und Acetylsalicylsäure ist; und

(c) gegebenenfalls Aufbewahren der gemäß Schritt (b) behandelten Prothese in einer sterilisierenden Lösung.

2. Verfahren nach Anspruch 1,
**dadurch gekennzeichnet,**
**daß** im Schritt (a) eine Lösung, die ein Gemisch von wenigstens drei verschiedenen Epoxidverbindungen enthält, verwendet wird.

3. Verfahren nach Anspruch 1 oder 2,
**dadurch gekennzeichnet,**
**daß** das Gemisch aus Epoxidverbindungen wenigstens eine nichtpolymere Epoxidverbindung mit zwei Epoxidgruppen umfaßt.

4. Verfahren nach einem der vorhergehenden Ansprüche, **dadurch gekennzeichnet,**
**daß** das Gemisch aus Epoxidverbindungen wenigstens eine polymere Epoxidverbindung mit zwei bis drei Epoxidgruppen und/oder eine Epoxidverbindung mit zwei bis drei Epoxidgruppen, wobei zwischen wenigstens zwei Epoxidgruppen ein gerad- oder verzweigkettige Kohlenwasserstoffkette mit wenigstens vier Kohlenstoffatomen angeordnet ist, umfaßt.

5. Verfahren nach einem der vorhergehenden Ansprüche, **dadurch gekennzeichnet,**
**daß** das Gemisch aus Epoxidverbindungen wenigstens eine Epoxidverbindung mit wenigstens drei Epoxidgruppen umfaßt.

6. Verfahren nach einem der vorherigen Ansprüche,
**dadurch gekennzeichnet,**
**daß** die Epoxidgruppe Bestandteil eines Glycidol-Restes ist.

7. Verfahren nach Anspruch 3,
**dadurch gekennzeichnet,**
**daß** die nichtpolymeren Epoxidverbindungen aus der Gruppe ausgewählt werden, die aus Alkylenglykoldiglycidylether, insbesondere Ethylenglykoldiglycidylether, Alkandioldiglycidylether, insbesondere 1,4-Butandioldiglycidylether, Polyalkoholdiglycidylether, insbesondere Glycerindiglycidylether, und Mischungen davon besteht.

8. Verfahren nach Anspruch 4,
**dadurch gekennzeichnet,**
**daß** die polymeren Epoxidverbindungen aus der Gruppe ausgewählt werden, die aus Polyalkylenglykoldiglycidylether, insbesondere Polyethylenglykoldiglycidylether, Polytetramethylenglykoldiglycidylether, Polypropylenglykoldiglycidylether und Mischungen davon besteht.

9. Verfahren nach Anspruch 4,
**dadurch gekennzeichnet,**
**daß** als Epoxidverbindung Alkandioldiglycidylether, insbesondere 1,6-Hexandioldiglycidylether, und/oder Dicarbonsäurediglycidylester verwendet werden.

10. Verfahren nach Anspruch 5,
**dadurch gekennzeichnet,**
**daß** die Epoxidverbindungen mit wenigstens drei Epoxidgruppen aus der Gruppe ausgewählt werden, die aus Polyalkoholpolyglycidylether, insbesondere Sorbitolpolyglycidylether, Glycerinpolyglycidylether, Pentaerythrolpolyglycidylether, Polysacharidpolyglycidylether, und Mischungen davon besteht.

11. Verfahren nach einem der vorhergehenden Ansprüche,
**dadurch gekennzeichnet,**
**daß** die biologische Prothese nach dem Behandeln gemäß Schritt (a) in Schritt (b) mit einer Lösung von Heparin und Acetylsalicylsäure ohne Verwendung zusätzlicher Reagenzien behandelt wird.

12. Verfahren nach Anspruch 11,
**dadurch gekennzeichnet,**
**daß** die biologische Prothese nach dem Behandeln mit Heparin und Acetylsalicylsäure gemäß Schritt (b) in destilliertem Wasser oder einer isotonischen Lösung, insbesondere einer 0,9 Gew.-%igen NaCl-Lösung, gespült wird und nachfolgend durch Behandeln mit einer Lösung einer beliebigen Epoxidverbindung sterilisiert wird.

**13.** Verfahren nach Anspruch 12,
**dadurch gekennzeichnet,**
**daß** die zur Sterilisierung verwendete Lösung eine Konzentration an der Epoxidverbindung von wenigstens 2 Gew.-% aufweist.

**14.** Verfahren nach einem der vorhergehenden Ansprüche,
**dadurch gekennzeichnet,**
**daß** die biologischen Prothese aus den Herzklappen von Säugetieren hergestellt wird.

**15.** Verfahren nach einem der vorhergehenden Ansprüche,
**dadurch gekennzeichnet,**
**daß** die biologischen Prothese aus Venenklappen oder Klappen enthaltenden Venensegmenten von Säugetieren hergestellt wird.

**16.** Verfahren nach einem der vorhergehenden Ansprüche,
**dadurch gekennzeichnet,**
**daß** die biologischen Prothese aus Arteriensegmenten von Säugetieren hergestellt wird.

**17.** Verfahren nach einem der vorhergehenden Ansprüche,
**dadurch gekennzeichnet,**
**daß** die biologischen Prothese aus membranösen Geweben, insbesondere Perikard oder harte Hirnhaut, von Säugetieren hergestellt wird.

**18.** Konservierte biologische Prothese,
**dadurch gekennzeichnet,**
**daß** die biologische Prothese nach einem Verfahren gemäß einem der Ansprüche 1 bis 17 hergestellt ist.

**19.** Konservierte biologische Prothese gemäß Anspruch 18,
**dadurch gekennzeichnet,**
**daß** die biologische Prothese eine Herzklappe, ein Arteriensegment, ein Venensegment, membranöses Gewebe, insbesondere Perikard oder harte Hirnhaut eines Säugetiers ist.

**20.** Konservierungslösung für biologische Prothesen,
**dadurch gekennzeichnet,**
**daß** die Konservierungslösung, bezogen auf die Gesamtmenge an Epoxidverbindungen,
40-80 Gew.-%, bevorzugt 50-70 Gew.-%, wenigstens einer nichtpolymeren Epoxidverbindung mit zwei Epoxidgruppen;
5-20 Gew.-%, bevorzugt 10-15 Gew.-%, wenigstens einer polymeren Epoxidverbindung mit zwei bis drei Epoxidgruppen und/oder wenigstens eine Epoxidverbindung mit zwei bis drei Epoxidgruppen, wobei zwischen wenigstens zwei Epoxidgruppen ein gerad- oder verzweigkettige Kohlenwasserstoffkette mit wenigstens vier Kohlenstoffatomen angeordnet ist,
15-45 Gew.-%, bevorzugt 20-25 Gew.-%, wenigstens einer Epoxidverbindung mit wenigstens drei Epoxidgruppen enthält, wobei die Gesamtmenge 100 Gew.-% ergibt.

**Claims**

**1.** A method of conserving biological prostheses, **characterised in that** the method includes the following steps:

(a) treating biological prostheses with a solution which contains a mixture of epoxide compounds which are at least in part of different lengths;
(b) treating the biological prosthesis treated in accordance with step
(a) with an antithrombotic-bearing solution, wherein the antithrombotic is a mixture of heparin and acetylsalicylic acid; and
(c) possibly storing the prosthesis treated in accordance with step (b) in a sterilising solution.

**2.** A method as set forth in claim 1 **characterised in that** step (a) involves using a solution which contains a mixture of at least three different epoxide compounds.

**3.** A method as set forth in claim 1 or claim 2 **characterised in that** the mixture of epoxide compounds includes at least one non-polymer epoxide compound with two epoxide groups.

**4.** A method as set forth in one of the preceding claims **characterised in that** the mixture of epoxide compounds includes at least one polymer epoxide compound with between two and three epoxide groups and/or an epoxide compound with between two and three epoxide groups, wherein arranged between at least two epoxide groups is a straight-chain or branched hydrocarbon chain with at least four carbon atoms.

**5.** A method as set forth in one of the preceding claims **characterised in that** the mixture of epoxide compounds includes at least one epoxide compound with at least three epoxide groups.

**6.** A method as set forth in one of the preceding claims **characterised in that** the epoxide group is a component of a glycidol residue.

**7.** A method as set forth in claim 3 **characterised in that** the non-polymer epoxide compounds are selected from the group which consists of alkylene glycol diglycidylether, in particular ethylene glycol diglycidylether, alkane diol diglycidylether, in particular butane-1,4-diol diglycidylether, polyalcohol diglycidylether, in particular glycerine diglycidylether, and mixtures thereof.

**8.** A method as set forth in claim 4 **characterised in that** the polymer epoxide compounds are selected from the group which consists of polyalkylene glycol diglycidylether, in particular polyethylene glycol diglycidylether, polytetramethylene glycol glycidylether, polypropylene glycol diglycidylether and mixtures thereof.

**9.** A method as set forth in claim 4 **characterised in that** the epoxide compound used is alkane diol diglycidylether, in particular hexane-1,6-diol diglycidylether, and/or dicarboxylic acid diglycidylester.

**10.** A method as set forth in claim 5 **characterised in that** the epoxide compounds with at least three epoxide groups are selected from the group which consists of polyalcohol polyglycidylether, in particular sorbitol polyglycidylether, glycerine polyglycidylether, pentaerythrol polyglycidylether, polysaccharide polyglycidylether and mixtures thereof.

**11.** A method as set forth in one of the preceding claims **characterised in that** after the treatment in accordance with step (a) in step (b) the biological prosthesis is treated with a solution of heparin and acetylsalicylic acid without using additional reagents.

**12.** A method as set forth in claim 11 **characterised in that** after the treatment with heparin and acetylsalicylic acid in accordance with step (b) the biological prosthesis is rinsed in distilled water or an isotonic solution, in particular a 0.9% by weight NaCl solution, and then sterilised by treatment with a solution of any epoxide compound.

**13.** A method as set forth in claim 12 **characterised in that** the solution used for sterilisation is of a concentration at the epoxide compound of at least 2% by weight.

**14.** A method as set forth in one of the preceding claims **characterised in that** the biological prosthesis is produced from the heart valves of mammals.

**15.** A method as set forth in one of the preceding claims **characterised in that** the biological prosthesis is produced from vein valves or valve-containing vein segments of mammals.

**16.** A method as set forth in one of the preceding claims **characterised in that** the biological prosthesis is produced from artery segments of mammals.

**17.** A method as set forth in one of the preceding claims **characterised in that** the biological prosthesis is produced from membranous tissues, in particular pericardium or hard meninges, of mammals.

**18.** A conserved biological prosthesis **characterised in that** the biological prosthesis is produced in accordance with a method as set forth in one of claims 1 through 17.

**19.** A conserved biological prosthesis as set forth in claim 18 **characterised in that** the biological prosthesis is a heart valve, an artery segment, a vein segment, membranous tissue, in particular pericardium or hard meninges of a mammal.

**20.** A conserving solution for biological prostheses **characterised in that** the conserving solution, in relation to the total amount of epoxide compounds, contains

40 - 80% by weight, preferably 50 - 70% by weight, of at least one non-polymer epoxide compound with two epoxide groups;

5 - 20% by weight, preferably 10 - 15% by weight, of at least one polymer epoxide compound with between two and three epoxide groups and/or at least one epoxide compound with between two and three epoxide groups, wherein arranged between at least two epoxide groups is a straight-chain or branched hydrocarbon chain with at least four carbon atoms; and

15 - 45% by weight, preferably 20 - 35% by weight, of at least one epoxide compound with at least three epoxide groups;

wherein the total amount is 100% by weight.

**Revendications**

**1.** Procédé pour la conservation de prothèses biologiques, **caractérisé en ce que** ce procédé comprend les étapes consistant à :

a) traiter des prothèses biologiques au moyen d'une solution contenant un mélange de composés époxydes présentant au moins partiellement des longueurs différentes ;

b) traiter la prothèse biologique traitée conformément à l'étape (a) au moyen d'une solution contenant un antithrombotique, le composé antithrombotique consistant en un mélange d'héparine et d'acide acétylsalicylique ; et

c) éventuellement conserver la prothèse traitée conformément à l'étape (b) dans une solution stérilisante.

**2.** Procédé selon la revendication 1, **caractérisé en ce que**, à l'étape (a) on utilise une solution contenant un mélange d'au moins trois composés époxydes différents.

**3.** Procédé selon la revendication 1 ou la revendication 2, **caractérisé en ce que** le mélange de composés époxydes comprend au moins un composé époxyde non polymère comportant deux groupes époxydes.

**4.** Procédé selon l'une quelconque des revendications précédentes, **caractérisé en ce que** le mélange de composés époxydes comprend au moins un composé époxyde polymère comportant deux à trois groupes époxydes et/ou un composé époxyde comportant deux à trois groupes époxydes dans lequel, entre au moins deux groupes époxydes, se trouve une chaîne hydrocarburée droite ou ramifiée comportant au moins quatre atomes de carbone.

**5.** Procédé selon l'une quelconque des revendications précédentes, **caractérisé en ce que** le mélange de composés époxydes contient au moins un composé époxyde comportant au moisn trois groupes époxydes.

**6.** Procédé selon l'une quelconque des revendications précédentes, **caractérisé en ce que** la portion constituant le groupe époxyde est un reste glycidol.

**7.** Procédé selon la revendication 3, **caractérisé en ce que** les composés époxydes non polymères sont sélectionnés dans le groupe constitué par les éthers diglycidyliques d'alkylène-glycols en particulier l'éther diglycidylique de l'éthylène glycol, l'éther diglycidylique d'un alkanediol en particulier l'éther diglycidylique du 1,4-butanediol, les éthers diglycidyliques de polyalcools et en particulier l'éther diglycidylique du glycérol et leurs mélanges.

**8.** Procédé selon la revendication 4, **caractérisé en ce que** les composés époxydes polymères sont sélectionnés dans le groupe constitué par les éthers diglycidyliques des polyalkylène-glycols, en particulier les éthers diglycidyliques des polyéthylène-glycols, les éthers diglycidyliques des polytétraméthylène-glycols, les éthers diglycidyliques des polypropylène-glycols et leurs mélanges.

9. Procédé selon la revendication 4, **caractérisé en ce qu'**on utilise comme composé époxyde un éther dyglicidylique d'alkanediol, en particulier l'éther dyglicidylique du 1,6-hexanediol et/ou l'ester dyglicidylique d'un acide dicarboxylique.

10. Procédé selon la revendication 5, **caractérisé en ce que** les composés époxydes comportant au moins trois groupes époxydes sont sélectionnés dans le groupe se composant des éthers polyglycidylique des polyalcools, en particulier les éthers polyglycidylique du sorbitol, les éthers polyglycidyliques du glycérol, les éthers polyglycidyliques du pentaérythritol, les éthers polyglycidyliques des polysaccharides et leurs mélanges.

11. Procédé selon l'une quelconque des revendications précédentes, **caractérisé en ce que** après le traitement conformément à l'étape (a), la prothèse biologique est traitée dans l'étape (b) au moyen d'une solution d'héparine et d'acide acétylsalicylique, sans utilisation de réactifs additionnels.

12. Procédé selon la revendication 11, **caractérisé en ce que** après le traitement par l'héparine et l'acide aétylsalicylique conformément à l'étape (b), la prothèse biologique est rincée dans de l'eau distillée ou une solution isotonique, en particulier dans une solution à 0,9% en poids de NaCl puis elle est stérilisée par traitement au moyen d'une solution dans un composé époxyde choisi.

13. Procédé selon la revendication 12, **caractérisé en ce que**, la solution utilisée pour la stérilisation présente une concentration en composé époxyde d'au moins 2% en poids.

14. Procédé selon l'une quelconque des revendications précédentes, **caractérisé en ce que** la prothèse biologique est préparée à partir de valvules cardiaques de mammifères.

15. Procédé selon l'une quelconque des revendications précédentes, **caractérisé en ce que** la prothèse biologique est préparée à partir de valvules de veines ou de segments de veines contenant des valvules d'animaux mammifères.

16. Procédé selon l'une quelconque des revendications précédentes, **caractérisé en ce que** la prothèse biologique est préparée à partir de segments d'artères de mammifères.

17. Procédé selon l'une quelconque des revendications précédentes, **caractérisé en ce que** la prothèse biologique est préparée à partir de tissu membraneux, en particulier de péricarde ou de méninge dure (dure-mère) de mammifères.

18. Prothèse biologique conservée, **caractérisée en ce que** la prothèse biologique est préparée conformément à un procédé selon l'une quelconque des revendications 1 à 17.

19. Prothèse biologique conservée selon la revendication 18, **caractérisée en ce que** la prothèse biologique consiste en une valvule cardiaque, un segment artériel, un segment veineux, un tissu membraneux en particulier du péricarde ou de la méninge dure (dure-mère) d'un mammifère.

20. Solution de conservation destinée à des prothèses biologiques, **caractérisée en ce que** la solution de conservation se compose, par rapport à la quantité totale de composés époxydes de:

   - 40-80% en poids, de préférence 60-70% en poids d'au moins un composé époxyde polymère comportant deux groupes époxydes,

   - 5-20% en poids, de préférence 10-15% en poids d'au moins un composé époxyde polymère comportant deux à trois groupes époxydes et/ou au moins un composé époxyde comportant deux à trois groupes époxydes, entre au moins deux groupes époxydes étant située une chaîne hydrocarburée droite ou ramifiée comportant au moins quatre atomes de carbone,

   - 15-45% en poids, de préférence 20-25% en poids d'au moins un composé époxyde comportant au moins trois groupes époxydes, ce qui donne la quantité totale de 100% en poids.